# EUROPEAN PATENT SPECIFICATION

(11) **EP 0 447 074 B1**
(45) Date of publication and mention of the grant of the patent: **13.12.1995**
(21) Application number: 91301690.3
(22) Date of filing: 01.03.1991
(51) Int. Cl.: C07D 251/34, C07C 269/02, C07C 271/28

(54) **Catalytic isocyanate trimerization and urethane formation**
Katalytische Trimerisation von Isocyanaten und Urethanbildung
Procédé catalysé pour la trimérisation d'isocyanates et pour la formation d'uréthanes

(30) Priority: 12.03.1990 JP 62427/90
(43) Date of publication of application: 18.09.1991
(73) Proprietor: ASAHI DENKA KOGYO KABUSHIKI KAISHA, Arakawa-ku Tokyo (JP)
(72) Inventor: Endo, Takeshi, Yokohama-shi, Kanagawa (JP); Nambu, Yoko c/o Asahi Denka Kogyo Kabushiki Kaisha, Arakawa-ku, Tokyo (JP)
(74) Representative: W.P. THOMPSON & CO.

(56) References cited:
- EP-A- 0 315 692
- EP-A- 0 339 396
- DE-B- 1 130 448
- FR-A- 2 256 153
- CHEMICAL ABSTRACTS, vol. 106, no. 9, 2 February 1987, Columbus, Ohio, US; abstract no. 67264Q, W. BRODA ET AL.: 'APPLICATIONS OF PHASE-TRANSFERCATALYSIS. PART 28. ONIUM SALT-CATALYZED PREPARATIONS OF ISOCYANURATES.' page 608;
- SYNTHESIS. no. 3, 1983, STUTTGART DE pages 169 - 184; G.G YAKOBSON ET AL.: 'ALKALI METAL FLUORIDES IN ORGANIC SYNTHESIS.'

## Description

The invention relates to catalytic isocyanate trimerization and urethane formation or urethanization. It further relates to a process for producing an isocyanate trimer and a process for producing an urethane.

Since an isocyanurate structure obtained by trimerizing an organic isocyanate brings about an improvement in the performance such as enhanced thermal resistance, flame retardancy, chemical resistance, etc., for polyurethane, coating material and the like, investigation has heretofore been made on a number of catalysts for isocyanate trimerization. For example, various metal salts of carboxylic acids, metal salts of DMF, tertiary amines, alcoholates of metals, etc., are effective catalysts for the above purpose. In addition, an epoxides/pyridine selectively forms a trimer. [see J. I. Jones et al., J. Chem. Soc., 4392(1957)].

However, the conventional catalysts for isocyanate trimerization as mentioned above have suffered the following disadvantages:
① insufficient activity necessitating severe reaction conditions,
② insufficient selectivity accompanied by the formation of by-products such as dimers, carbodiimides, etc.,
③ difficulty in eliminating high-boiling additives such as DMF, and
④ inevitable side reactions due to the attack by the active species of a catalyst upon the functional groups such as ester, silyl ether, etc., in the system containing such a group.

Shiomura et al. have recently reported in JP-A-287773/1988 and JP-A-290871/1988, and elsewhere, the trimerization of organic isocyanates in the presence of potassium fluoride alone or together with polyethylene oxide as a phase-transfer catalyst. However, no sufficient activity can be obtained in the presence of the single catalyst.

It has been a usual practice in the production of a polyurethane having an isocyanurate structure to use a complex system containing a trimerization catalyst and a urethanization catalyst, for example, a catalyst mixture of potassium acetate and a tertiary amine.

On the other hand, the present inventors have already found the selective ring-opening reaction of a glycidyl ether with an aryl silyl ether using cesium fluoride as the catalyst (see JP-A-57981/1988; Y. Nambu, T. Endo, Tetrahedron Lett.,31, 1723(1990)) and are advancing the investigation on a variety of organic reactions by the use of fluoride catalysts.

An object of the present invention is to provide a process for a catalytic isocyanate trimerization and catalytic urethanization, each being improved against the above-mentioned disadvantages.

In the process for producing an isocyanate trimer according to the present invention, cesium fluoride is used as the catalyst in an amount of from 0.001 to 0.01 equivalent, on the basis of the isocyanate and the process is carried out in the absence of a solvent or in an aprotic polar solvent.

Further, in the process for producing a urethane according to the present invention, cesium fluoride is used as the catalyst in an amount of from 0.001 to 0.1 equivalent, on the basis of the isocyanate and the process is carried out in the absence of a solvent or in an aprotic polar solvent.

The cesium fluoride catalyst according to the present invention is preferably used in a sufficiently dried state.

The amount of the catalyst to be used in the present invention ranges more desirably from 0.005 to 0.05 equivalent on the basis of the isocyanate.

The isocyanate to be used in the trimerization or urethanization according to the present invention is exemplified by those having a substituted or unsubstituted alkyl or aryl group. The reaction proceeds selectively even when the isocyanate has a halide, ester, isocyanate, alkyl ester, aryl ester, silyl ether, ether, amide, ketone, unsaturated carbon, nitro, or the like group as the substituent.

The compound having a hydroxyl group which is used in the urethanization according to the present invention is exemplified by substituted or unsubstituted, monofunctional or multifunctional, straight-chain or branched alcohol and substituted or unsubstituted aromatic compound having a hydroxyl group.

The process for producing the isocyanate trimer or urethane is put into practice without any solvent or in an aprotic polar solvent. Examples of the aprotic polar solvent include acetonitrile, acetone, DMF, and THF. The proper amount of the solvent to be used ranges from 0.3 to 2 equivalents on the basis of the isocyanate.

In what follows the preferred embodiments of the present invention will be described.

The trimerization may be carried out by reacting an organic isocyanate at room temperature to 150 °C for one minute to 2 hours without any solvent by using 0.005 to 0.02 equivalent of cesium fluoride as the catalyst. After the reaction, unreacted isocyanate is removed under reduced pressure, the reaction product is dissolved in methylene chloride, the catalyst is separated by filtration and the methylene chloride solvent is distilled away to obtain an isocyanurate corresponding to the starting isocyanate.

The urethanization and trimerization accompanied by urethanization are carried out by reacting an organic isocyanate with 0.1 to 1 equivalent of an organic hydroxylic compound under the almost same conditions as those described above to obtain a urethane and an isocyanurate corresponding to the starting isocyanate.

According to the present invention, cesium fluoride is a novel and highly active catalyst useful for isocyanate trimerization and urethanization.

The use of the catalyst according to the present invention enables the trimerization of organic isocyanates to proceed under milder conditions than those which obtain with conventional catalysts to thereby obtain isocyanurate derivatives each having a high purity. Furthermore, the catalyst according to the present invention exhibits such a high selectivity for the trimerization of isocyanates that it can be applied to the synthesis of novel isocyanurate derivatives having a variety of reactive groups.

Moreover, the catalyst according to the present invention is effective for the reaction of an organic isocyanate with a compound having a hydroxyl group to produce a urethane and an isocyanate quantitatively in the ratio corresponding to the feed ratio of the above two raw materials under mild reaction conditions. Thus, the catalysts according to the present invention are highly effective for the production of a polyurethane having excellent performance and, furthermore, exhibit high activity for the reaction of isocyanates with phenols of low reactivity as well as enabling themselves to be utilized for synthesizing masked isocyanates.

The present invention will now be described in more detail by the following Examples, but shall not be limited thereto.

### Example 1

1.28 g (8.4 mmol) of cesium fluoride was weighed into a flask and dried under a reduced pressure at 130 °C for 30 minutes. When 50 g (0.42 mol) of phenyl isocyanate was added thereto with stirring and heating at 130 °C, the mixture in the system solidified after one minute. After 4 minutes of further heating, an unreacted phenyl isocyanate was removed under a reduced pressure, the residue was dissolved in methylene chloride, the catalyst was separated by filtration and thereafter the solvent was distilled away to obtain 45 g of triphenyl isocyanurate at a yield of 90.5 %. The structure of the product was confirmed by the melting point (285 °C), NMR and IR. No formation of a dimer was observed.

### Example 2

The same reaction system as that in Example 1 was allowed to react at room temperature for 20 minutes, followed by the same treatment as that in Example 1 to obtain 45 g of a solid. By washing the solid with ether 40.1 g of triphenyl isocyanurate was obtained at a yield of 80.2 % as the ether-insoluble along with 4.4 g of a dimer at a yield of 8.8 % as the ether-soluble.

### Comparative Examples 1 to 3

The procedure of Example 2 was repeated with various catalysts (2 mol%) listed in Table 1 instead of cesium fluoride to conduct reaction.

Table 1 shows the results of Comparative Examples 1 to 3 as well as those of Examples 1 to 2. As obvious from Table 1, the use of cesium fluoride as the catalyst enabled the trimer to be produced at a high yield in a short time.

**Table 1**

| | Catalyst | Reaction temp. | Reaction time(min) | Yield (%) | |
|---|---|---|---|---|---|
| | | | | Trimer | Dimer |
| Example 1 | CsF | 130 °C | 5 | 90.5 | 0 |
| Example 2 | CsF | room temp. | 20 | 80.2 | 8.8 |
| Comp. Ex. 1 | AcOK | do. | 20 | 0 | 0 |
| Comp. Ex. 2 | Et₃N | do. | 20 | 0 | 0 |
| Comp. Ex. 3 | KF | do. | 20 | 1.4 | |

### Remarks:

Bu, Ac and Et indicate butyl, acetyl and ethyl, respectively.

### Example 3

In the same manner as that in Example 1, 4-allyloxyphenyl isocyanate was heated at 130 °C for 10 minutes with stirring in the presence of cesium fluoride as the catalyst (2 mol%), and the resultant solidified product was recrystallized from ethanol to obtain tris(4-allyloxyphenyl) isocyanurate at a yield of 85.4 %.
Melting point: 218-220 °C

| Elemental analysis: | | | |
|---|---|---|---|
| | C | H | N |
| Found (%): | 68.79 | 5.31 | 8.06 |
| Calculated (%): | 68.56 | 5.13 | 8.00 |

### Example 4

In the same manner as that in Example 1, 4-nitrophenyl isocyanate was heated at 150 °C for 1 hour with stirring in the presence of cesium fluoride as the catalyst (2 mol%), and tris(4-nitrophenyl) isocyanurate was obtained at a yield of 75 %.
Melting point: 275-277 °C

| Elemental analysis result: | | | |
|---|---|---|---|
| | C | H | N |
| Found (%): | 51.35 | 2.63 | 17.35 |
| Calculated (%): | 51.22 | 2.46 | 17.07 |

### Examples 5 and 6

In the same manner as that in Example 1, n-butyl isocyanate was reacted under the reaction conditions specified in Table 2 in the presence of cesium fluoride alone or the cesium fluoride/trimethylsilyl phenoxide system as the catalyst (2 mol%), and tributyl isocyanurate was obtained at each yield given in Table 2 even for the above alkyl isocyanate with low reactivity:
¹H-NMR(CDCl₃) δ : 0.8 - 2.0 ppm [m, 21H, CH₃(CH₂)₂], 3.88[t, 6H, CH₂N]

**Table 2**

| | Catalyst | Reaction temp. | Reaction time | Yield (%) |
|---|---|---|---|---|
| Example 5 | CsF | 130 °C | 60 min | 62.0 |
| Example 6 | CsF/PhOSiMe₃ | 130 °C | 10 min | 95.0 |

As shown in Table 2, the combined use of cesium fluoride with alkyl or aryl silyl ether can enhance the yield of the respective product.

### Example 7

1.28 g (8.4 mmol) of cesium fluoride was weighed into a flask and dried under a reduced pressure at 130 °C for 30 minutes. When 50 g (0.42 mol) of phenyl isocyanate and 6.43 ml (0.084 mol) of isopropanol were added thereto at room temperature with stirring, much heat was evolved in the mixture resulting in solidification in 20 seconds. After 2 minutes, an unreacted raw material was removed from the system under a reduced pressure to obtain 52.0 g of a solid at a yield of 94.5 %. From the ether-soluble of the solid, 13.1 g of isopropyl N-phenylcarbamate was obtained as the urethanization product at a yield of 88.0 % based on the starting isopropanol. The chemical structure of the product having a melting point of 83 to 84 °C was confirmed by ¹H-NMR and IR. In addition 38.9 g of triphenyl isocyanurate was obtained as the trimerization product as the ether-insoluble.

### Comparative Example 4

A liquid mixture of 50 g (0.42 mol) of phenyl isocyanate and 6.43 ml (0.084 mol) of isopropanol was stirred at room temperature for 20 minutes and thereafter an unreacted raw material was distilled away under a reduced pressure to obtain 16.1 % of isopropyl N-phenylcarbamate.

### Example 8

1.28 g (8.4 mmol) of cesium fluoride was weighed into a flask and dried under a reduced pressure at 130 °C for 30 minutes. 50 g (0.42 mol) of phenyl isocyanate and 39.5 g (0.42 mol) of phenol were added thereto with stirring at 105 °C for 10 minutes, followed by the removal of an unreacted raw material at a reduced pressure. Thus, 88.3 g of phenyl N-phenylcarbamate having a melting point of 123 to 124 °C was obtained at a yield of 93.1 %.

### Comparative Example 5

The procedure of Example 8 was repeated except that no catalyst was added to the reaction system. As a result, 8.3 g of phenyl N-phenylcarbamate was obtained at a yield of 9.3 %.

## Claims

1. A process for producing an isocyanate trimer by isocyanate trimerisation, characterised by the use, in an amount of from 0.001 to 0.1 equivalent, on the basis of the isocyanate, of cesium fluoride as a catalyst, the process being carried out in the absence of a solvent or in an aprotic polar solvent.

2. A process for producing a urethane from reactants including an isocyanate, characterised by the use, in an amount of from 0.001 to 0.1 equivalent, on the basis of the isocyanate, of cesium fluoride as a catalyst, carried out in the absence of a solvent or in an aprotic polar solvent.

3. A process according to either preceding claim, in which the isocyanate has a substituted or unsubstituted alkyl or aryl group.

4. A process according to claim 1 or 2, in which the isocyanate is halide, ester, isocyanate, alkyl ester, aryl ester, silyl ether, ether, amide, ketone, unsaturated carbon or nitro substituted.

5. A process according to claim 2, in which the hydroxy compound taking place in the urethanization is a substituted or unsubstituted, monofunctional or multifunctional, straight-chain or branched-chain alcohol or a substituted or unsubstituted aromatic compound having a hydroxyl group.

6. A process according to any preceding claim, in which the catalyst is used in an amount of from 0.005 to 0.05 equivalent, on the basis of the isocyanate.

7. A process according to any preceding claim, carried out in an aprotic solvent which is used in an amount of 0.3 to 2 equivalents, on the basis of the isocyanate.

8. A process according to claim 1, carried at room temperature to 150°C for one minute to 2 hours in the absence of a solvent and using cesium fluoride in an amount of 0.005 to 0.02 equivalent, on the basis of the isocyanate.

## Patentansprüche

1. Verfahren zum Herstellen eines Isocyanattrimers durch Isocyanattrimerisation, das durch die Verwendung von Cesiumfluorid als Katalysator in einer Menge von 0,001 bis 0,1 Äquivalenten bezogen auf das Isocyanat gekennzeichnet ist, wobei das Verfahren in Abwesenheit eines Lösungsmittels oder in einem aprotischen polaren Lösungsmittel durchgeführt wird.

2. Verfahren zum Herstellen eines Urethans aus Reaktanden, die ein Isocyanat einschließen, das durch die Verwendung von Cesiumfluorid als Katalysator in einer Menge von 0,001 bis 0,1 Äquivalenten bezogen auf das Isocyanat gekennzeichnet ist und in Abwesenheit eines Lösungsmittels oder in einem aprotischen polaren Lösungsmittel durchgeführt wird.

3. Verfahren gemäß einem vorangehenden Anspruch, bei dem das Isocyanat eine substituierte oder unsubstituierte Alkyl- oder Arylgruppe besitzt.

4. Verfahren gemäß Anspruch 1 oder 2, bei dem das Isocyanat durch Halogenid, Ester, Isocyanat, Alkylester, Arylester, Silylether, Ether, Amid, Keton, ungesättigten Kohlenstoff oder Nitro substituiert ist.

5. Verfahren gemäß Anspruch 2, bei dem die an der Urethanbildung teilnehmende Hydroxyverbindung ein substituierter oder unsubstituierter, monofunktioneller oder multifunktioneller, geradkettiger oder verzweigtkettiger Alkohol oder eine substituierte oder unsubstituierte aromatische Verbindung mit einer Hydroxylgruppe ist.

6. Verfahren gemäß einem vorangehenden Anspruch, bei dem der Katalysator in einer Menge von 0,005 bis 0,05 Äquivalenten bezogen auf das Isocyanat verwendet wird.

7. Verfahren gemäß einem vorangehenden Anspruch, das in einem aprotischen Lösungsmittel durchgeführt wird, welches in einer Menge von 0,3 bis 2 Äquivalenten bezogen auf das Isocyanat verwendet wird.

8. Verfahren gemäß Anspruch 1, das eine Minute bis 2 Stunden bei Raumtemperatur bis 150°C in Abwesenheit eines Lösungsmittels und unter Verwenden von Cesiumfluorid in einer Menge von 0,005 bis 0,02 Äquivalenten bezogen auf das Isocyanat durchgeführt wird.

## Revendications

1. Procédé de préparation d'un trimère d'isocyanate par trimérisation d'isocyanate, caractérisé par l'utilisation de fluorure de césium en tant que catalyseur, en une quantité de 0,001 à 0,1 équivalent, sur la base de l'isocyanate, le procédé étant réalisé en l'absence de solvant ou dans un solvant polaire aprotique.

2. Procédé de préparation d'un uréthane à partir de réactifs comprenant un isocyanate, caractérisé par l'utilisation de fluorure de césium en tant que catalyseur, en une quantité de 0,001 à 0,1 équivalent, sur la base de l'isocyanate, le procédé étant réalisé en l'absence de solvant ou dans un solvant polaire aprotique.

3. Procédé selon l'une quelconque des revendications précédentes, dans lequel l'isocyanate possède un groupe alkyle ou aryle substitué ou non.

4. Procédé selon la revendication 1 ou 2, dans lequel l'isocyanate est substitué par un halogénure, un ester, un isocyanate, un ester d'alkyle, un ester d'aryle, un éther de silyle, un éther, une amide, une cétone, un carbone insaturé ou un nitro.

5. Procédé selon la revendication 2, dans lequel le composé hydroxy prenant place dans l'uréthanisation est un alcool à chaîne droite ou ramifiée, mono- ou multi-fonctionnel, substitué ou non, ou un composé aromatique substitué ou non possédant un groupe hydroxy.

6. Procédé selon l'une quelconque des revendications précédentes, dans lequel le catalyseur est utilisé en une quantité de 0,005 à 0,05 équivalent, sur la base de l'isocyanate.

7. Procédé selon l'une quelconque des revendications précédentes, réalisé dans un solvant aprotique utilisé en une quantité de 0,3 à 2 équivalents, sur la base de l'isocyanate.

8. Procédé selon la revendication 1, réalisé de la température ambiante à 150°C pendant une minute à deux heures, en l'absence d'un solvant et en utilisant du fluorure de césium en une quantité de 0,005 à 0,02 équivalent, sur la base de l'isocyanate.
